# EUROPEAN PATENT SPECIFICATION

(11) **EP 1 820 532 B1**
(45) Date of publication and mention of the grant of the patent: **05.11.2008**
(21) Application number: 06003467.5
(22) Date of filing: 21.02.2006
(51) Int. Cl.: A61M 39/26, A61M 39/02

(54) **Device with a bi-directional valve for injecting medicine and drawing body fluid without using a needle**
Vorrichtung mit einem Zweiwegventil zum Einspritzen von medizinischen Lösungen und Entnehmen von Flüssigkeit ohne Verwendung einer Nadel
Dispositif comprenant une valve bidirectionelle pour l'injection de solutions médicales et le prélèvement de fluides sans l'utilisation d'une aiguille

(43) Date of publication of application: 22.08.2007
(73) Proprietor: Tsai, Hsi-Chin, Shulin City Taipei Hsien (TW)
(72) Inventor: Tsai, Hsi-Chin, Shulin City Taipei Hsien (TW)
(74) Representative: Hauck Patent- und Rechtsanwälte

(56) References cited:
- EP-A- 0 791 371
- US-A- 5 782 816
- US-A- 5 806 551
- US-A1- 2005 010 177

## Description

### 1. Field of the invention

The present invention relates to a device, and especially to a device with a bi-directional valve for injecting medicine and drawing body fluid without using a needle.

### 2. Description of Related Art

When a patient is injected the nutrients or medical solution or took small amount of blood, he must prick with a needle. However, to reduce traumas resulting from pricking with a needle an injection joint is used if he is injected with nutrients or medical solution or took small amount of blood repeatedly in the short time. Furthermore, when a patient is put on a drip, nutrients or other medical solution may be injected into the patient through a drip tube assembly that controls the infusion speed and communicates with the injection joint. A syringe with a needle punctures the injection joint to inject the nutrients or medical solution or take small amount of blood so that a contaminated needle may contaminate the injection joint. Furthermore, the contaminated needle may transfer infectious material to the nursing staff if the nursing staff prick with the contaminated needle. Therefore, it is known from US 2005/0010177 that a conventional injection joint can be injected the nutrients or medical solution without using the syringe with the needle.

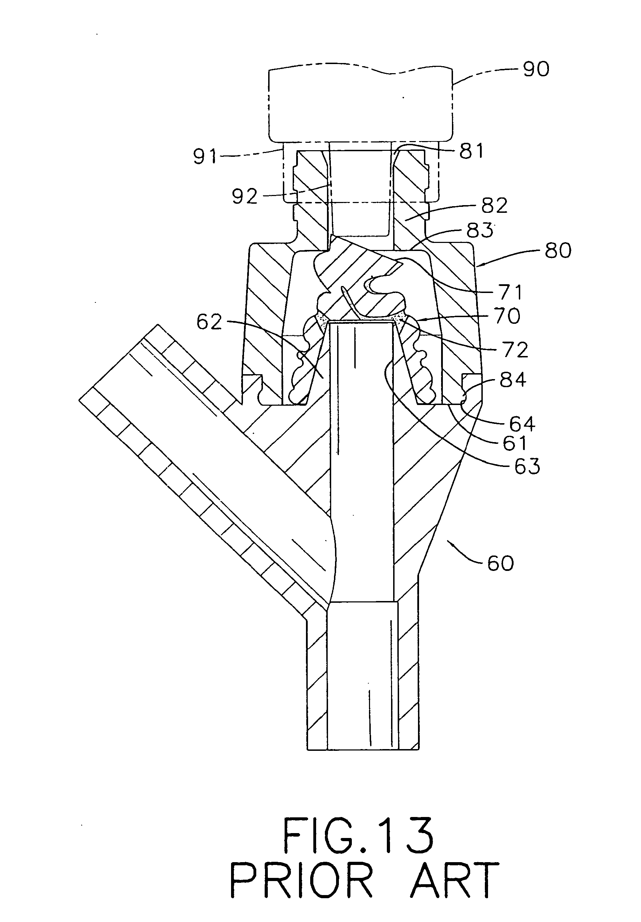

With reference to Fig. 13, the conventional injection joint has a connector (60), a resilient plug (70) and a cap (80).

The connector (60) is Y shape and has a top, a plane (61), a protrusion (62), a passage (63) and a sidewall. The plane (61) is formed on the top of the connector (60) and has a center. The protrusion (62) protrudes from the center of the plane (61) and has a top. The passage (63) is formed in the protrusion (62) and extends through the connector (60). The sidewall is formed on the top of the connector and around the plane (62) and has a groove (64) and an inner face. The groove (64) is formed around the inner surface of the sidewall.

The resilient plug (70) is mounted around the protrusion (62) and has a top, a bottom, a flange (71), a cavity (72) and a bottom tube. The flange (71) is formed on the top of the resilient plug (70) and has a top. The cavity (72) is formed inside the resilient plug (70) and the medical solution can flow through the cavity (72) into the passage (61). The bottom tube is formed on the bottom of the resilient plug (70) and is mounted around the protrusion (62).

The cap (80) is mounted around the resilient plug (70) and has a top, an opening (81), a neck (82), a shoulder (83) and a mounting ring (84). The opening (81) is formed in the top and corresponds to the top of the flange (71). The neck (82) is formed between the opening (81) and the shoulder (83) and abuts with the flange (71) so the flange (71) closes the opening (81) in the neck (82).

A syringe (90) without a needle has an inner threaded opening (91), an inner tube (92) and a plunger. The inner tube (92) protrudes from the inner threaded opening (91).

When the inner threaded opening (91) of the syringe (90) is screwed onto the neck (82) of the cap (80), the inner tube (92) abuts and pushes the top of the resilient plug (70) to open the opening (81). After pushing the plunger to inject nutrients or other medical solution, the flange (71) of the resilient plug collapses and the solution flow through the cavity (72) into the passage (61). However, the resilient plug (70) cannot return to an original position because the top of the flange (71) of the resilient plug (70) wedges in the shoulder (83) of the cap (80).

The main objective of the present invention is to provide a device with a bi-directional valve for injecting medicine and drawing blood without using a needle to improve the resilient plug when the resilient plug returns to original position without wedging.

To achieve the objective, a safety lancet device in accordance with the present invention comprises a connector with a resilient plug and a cap with an opening. The resilient plug is mounted in the cap and has a neck, a flange and multiple holes. The neck has a top which the flange is formed on and closes the opening of the cap. The multiple holes are formed on the resilient plug. The cap has a concave inner surface of a shoulder. After pushing a plunger of a syringe without a needle, the solution flows through the multiple holes. The flange and the neck can return to close the opening because the concave inner surface of the shoulder and the smooth edge of the flange reduce the friction.

### IN THE DRAWINGS:

Fig. 1 is an exploded perspective view of a first embodiment of a device with a bi-directional valve for injecting medicine and drawing body fluid without using a needle in accordance with the present invention;
Fig. 2 is a side view in partial section of the first embodiment of the device in Fig. 1;
Fig. 3 is a bottom view of a resilient plug in the device in Fig. 1
Fig. 4 is a side view in partial section of the resilient plug in the device in Fig. 1;
Fig. 5 is an operational side view in partial section of the device in Fig. 1 when a syringe is screwed onto a threaded neck of a cap;
Fig. 6 is an operational side view in partial section of the device in Fig. 1 when solution is injected into the device;
Fig. 7 is an exploded perspective view of a second embodiment of a device with a bi-directional valve for injecting medicine and drawing body fluid without using a needle in accordance with the present invention;
Fig. 8 is a side view in partial section of the second embodiment of the device in Fig. 7;
Fig. 9 is a side view in partial section of a third embodiment of a device with a bi-directional valve for injecting medicine and drawing body fluid without using a needle in accordance with the present invention;
Fig. 10 is a side view in partial section of a fourth embodiment of a device with a bi-directional valve for injecting medicine and drawing body fluid without using a needle in accordance with the present invention;
Fig. 11 is a side view in partial section of a fifth embodiment of a device with a bi-directional valve for injecting medicine and drawing body fluid without using a needle in accordance with the present invention;
Fig. 12 is a side view in partial section of a sixth embodiment of a device with a bi-directional valve for injecting medicine and drawing body fluid without using a needle in accordance with the present invention; and
Fig. 13 is an operational side view in partial section of the injection joint in accordance with the prior art when a syringe is screwed onto a neck of a cap.

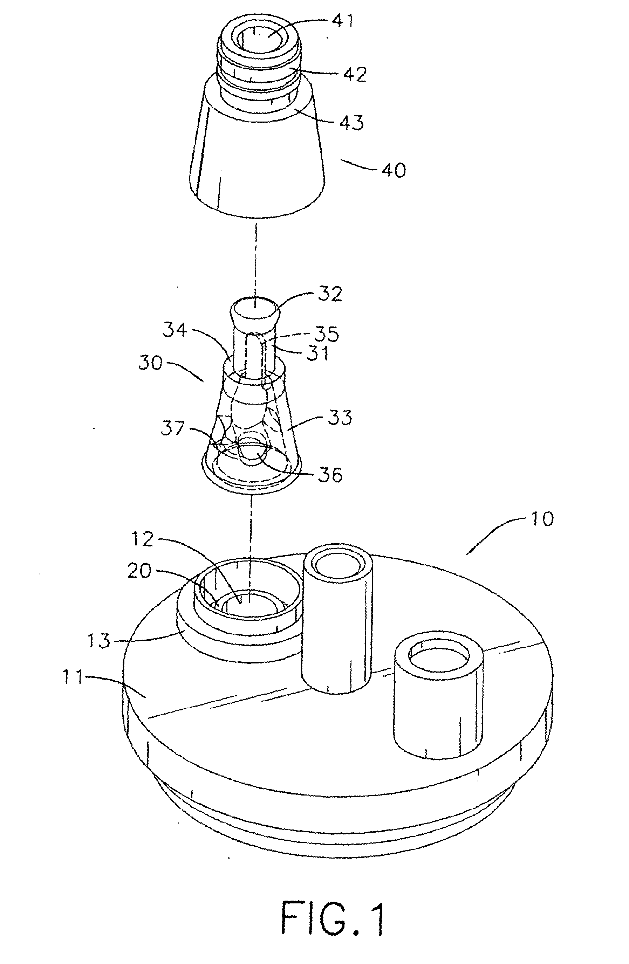

With reference of Figs. 1, 2 and 8 to 12, a device with a bi-directional valve for injecting medicine and drawing body fluid without using a needle in accordance with the present invention comprises a connector (10, 10a, 10b, 10c, 10d, 10e), an optional protruding ring (20, 20a), a resilient plug (30) and a cap (40).

The connector (10, 10a, 10b, 10c, 10d, 10e) comprises a top, a bottom, a plane (11), a passage (12) and a sidewall (13). The plane (11) is formed on the top of the connector (10, 10a, 10b, 10c, 10d, 10e) and has a center. The passage (12) is formed through the connector (10, 10a, 10b, 10c, 10d, 10e) and has a top end opening in the plane (11). The sidewall (13) is formed on the top of the connector (10, 10a, 10b, 10c, 10d, 10e) to surround the top end of the passage (12) and has a top, an outer face and an inner face.

The protruding ring (20, 20a) may be formed on or mounted on the plane (11) and around the top end of the passage (12).

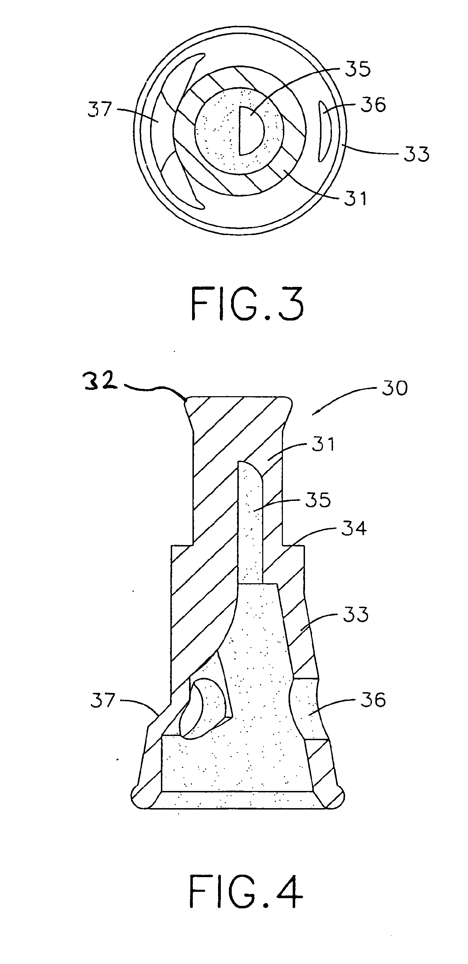

With reference to Figs. 3 and 4, the resilient plug (30) is mounted on the plane (11) and around the protruding ring (20, 20a) and has a top, a bottom, a neck (31), a flange (32), a bottom tube (33), an annular shoulder (34), an inner cavity (35) and multiple holes (36). The neck (31) is cylindrical and has a top and a bottom. The flange (32) is formed on the top of the neck (31) and has a top and a smooth edge. The bottom tube (33) extends downward from the bottom of the neck (31) has a diameter larger than that of the neck (31) and has a bottom, an outer surface, an inner surface and a horizontal constriction (37). The horizontal constriction (37) is formed on half of the outer surface of the bottom tube (33) so the resilient plug (30) topples over from the horizontal constriction (37) when the resilient plug (30) is pushed. The annular shoulder (34) is formed between the neck (31) and the bottom tube (33). The inner cavity (35) is formed in the neck (31) and communicates with the bottom tube (33). The multiple holes (36) are formed through the bottom tube (33) near the bottom so the fluid can flow through the multiple holes (36).

The cap (40) is mounted on the resilient plug (30) and has a top, a bottom, an inner surface, an opening (41), a threaded neck (42), a shoulder (43), a concave inner surface (45) and an optional mounting ring (44). The threaded neck (42) is formed on and extends from the top and has a diameter smaller than that of the cap (40) so as to define a shoulder (43) between the threaded neck (42) and the top of the cap (40). The opening (41) is formed through the threaded neck (42) and corresponds to and holds the top of the flange (32) inside so the flange (32) closes the opening (41). The concave inner surface (45) is defined in the inner surface of the cap (40) at a joint between the cap (40) and the threaded neck (42). The mounting ring (44) is formed near the bottom on the cap (40) and mounted securely on or in the sidewall (13) to hold the cap (40) in the connector (10, 10a, 10b, 10c, 10d, 10e) and around the resilient plug (30).

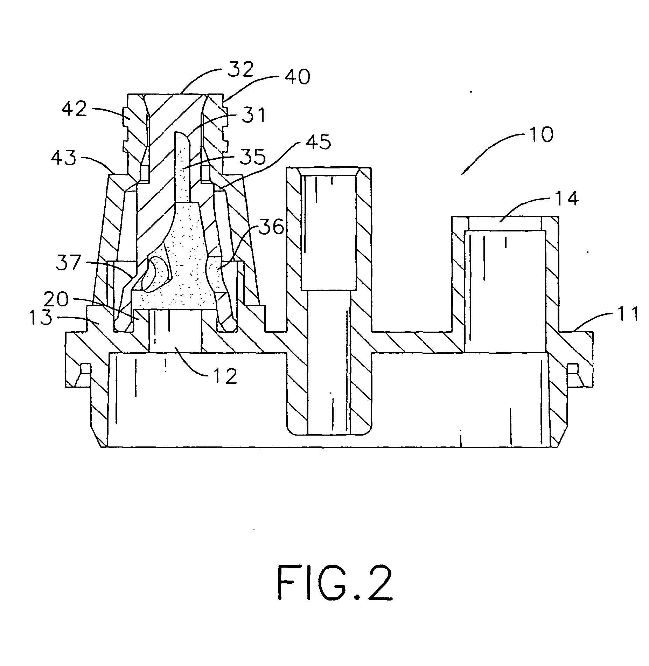

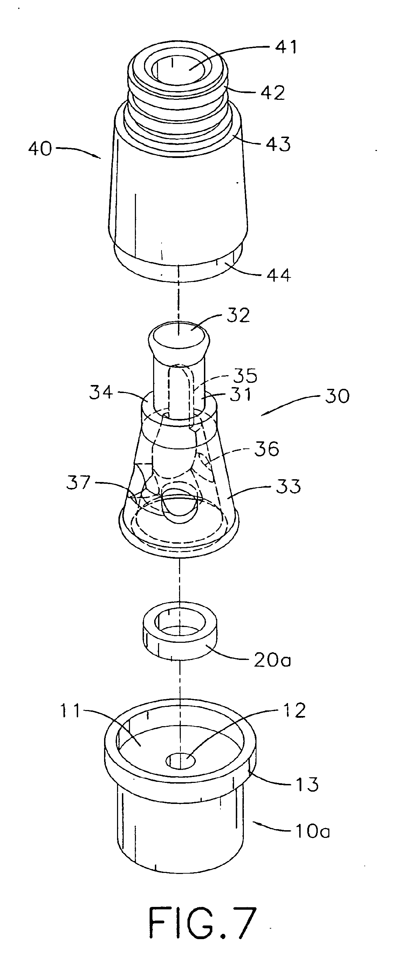

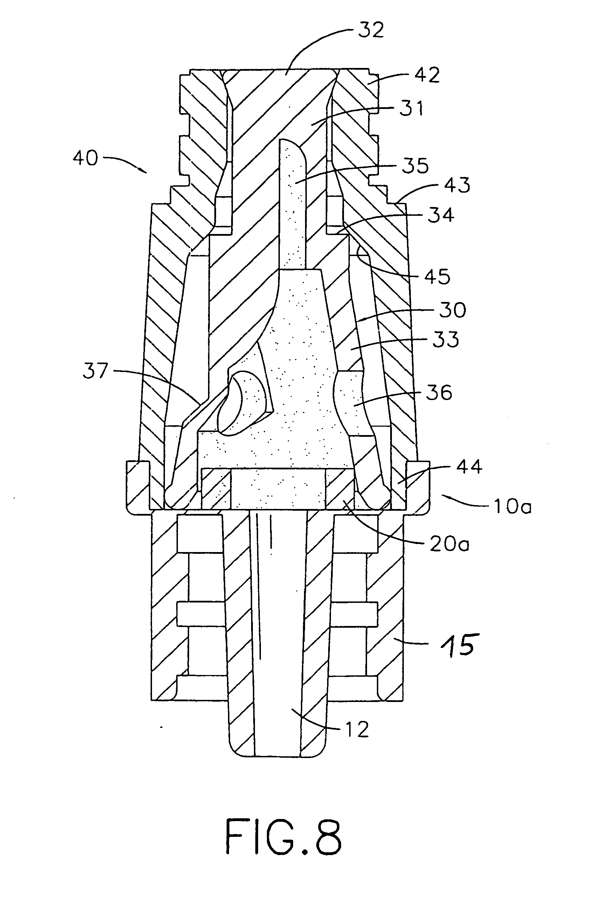

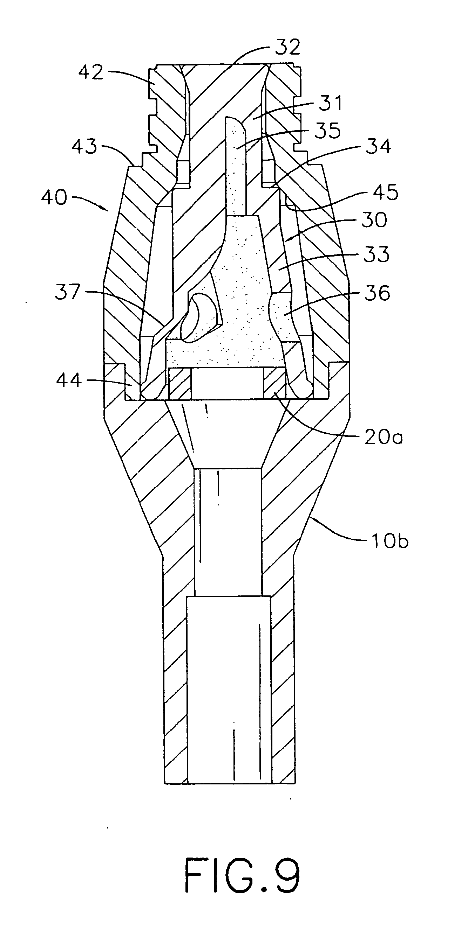

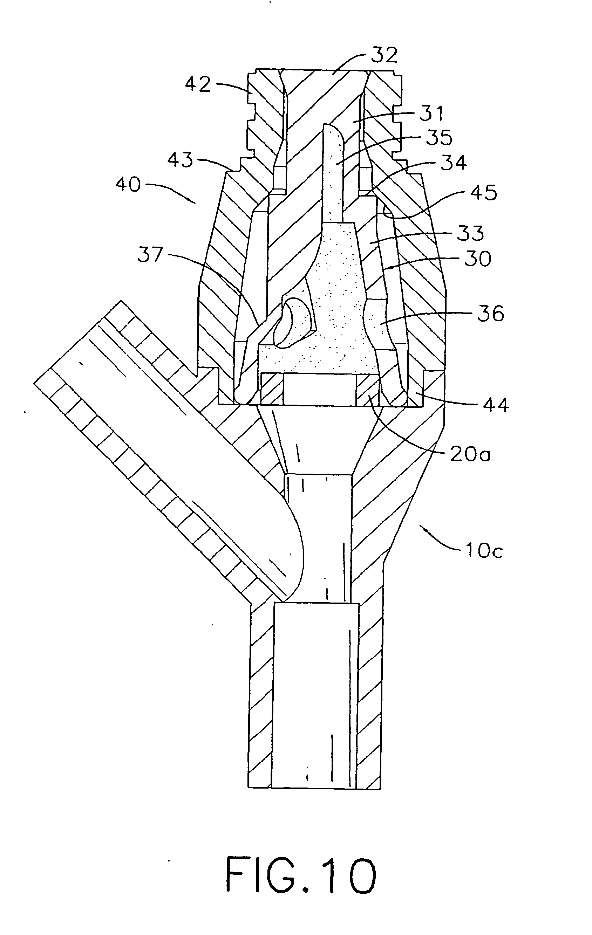

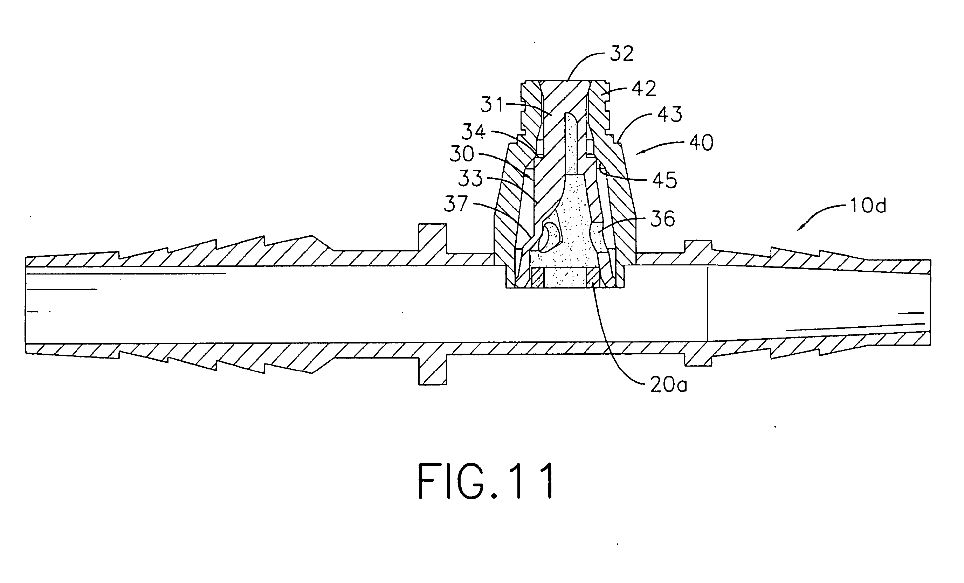

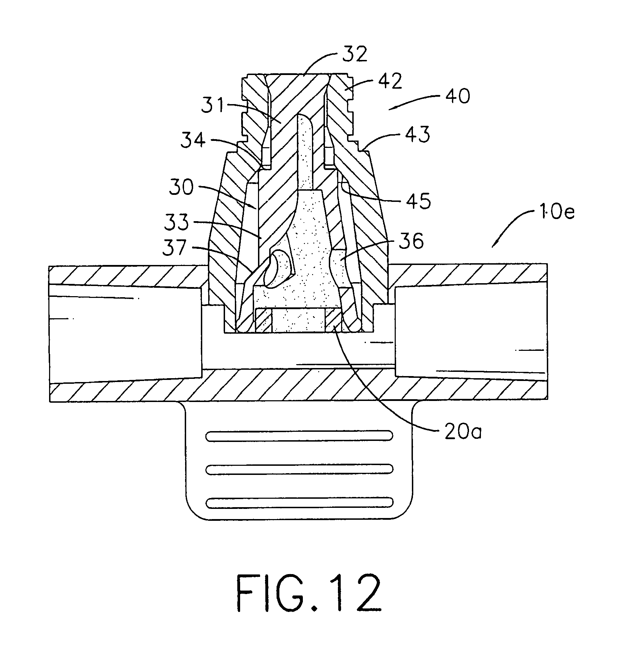

In the first embodiment, with reference to Fig. 1 and 2, the connector (10) is connected a container and further comprises multiple passages (14) to connect another tube. Furthermore, the protruding ring (20) is formed on the plane (11) and around the top end of the passage (12) to avoid that the inner surface of the bottom tube (33) of the resilient plug (30) wedge into the passage (12). In a second to a sixth embodiment, with reference to Figs. 7 to 12, the connector (10a, 10b, 10c, 10d, 10e) are tubular and the protruding ring (20a) are mounted on the plane (11). With reference to Fig. 8, the connector (10a) further comprises an inner threaded joint (15) formed under the plane (11) to connect to a tube. The connector (10b) as shown in Fig. 9 can be mounted on the bi-direction catheter. The connector (10c) as shown in Fig. 10 is Y-shaped. The connectors (10d, 10e) as shown in Figs. 11 and 12, are T-shaped. The connector (10d) can be used to a urine sampling adapter. The connector (10e) can be used to a sampling adapter.

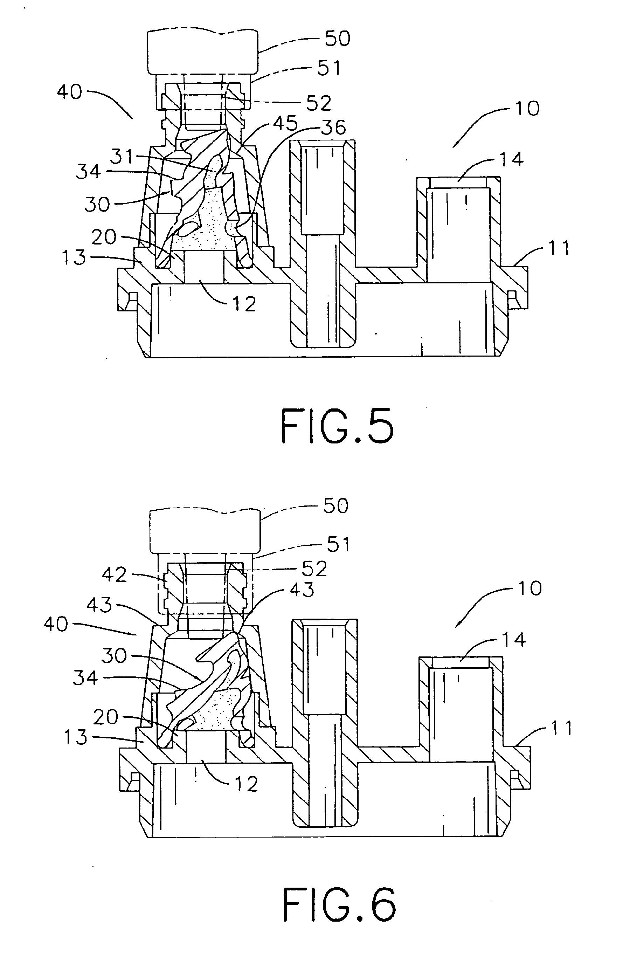

With reference to Fig. 5 and 6, a syringe (50) without a needle has an inner threaded opening (51), an inner tube (52) and a plunger. The inner tube (52) protrudes from the inner threaded opening (51).

When the inner threaded opening (51) of the syringe (50) is screwed onto the threaded neck (42) on the cap (40), the inner tube (52) abuts against and pushes the top of the resilient plug (30). Consequently, the flange (32) and the neck (31) of the resilient plug (30) are compressed and deformed to open the opening (41). While pushing the plunger to inject solution into the connector (10, 10a, 10b, 10c) or pulling the plunger to absorb fluid through the connector (10d, 10e), the solution flows through the multiple holes (36) into the passage (61) or the fluid flows through the multiple holes (36) into the syringe (50). The flange (32) and the neck (31) return to close the opening (41) of the cap (40) when the syringe is removed from the cap (40). Furthermore, the protruding ring (20) keeps the inner surface of the bottom tube (33) of the resilient plug (30) from wedging into the passage (12). With the arrangement of the concave inner surface (45), the shoulder (34) on the resilient plug (30) is kept from be blocked by concave inner surface (45), so the resilient plug (30) can return to the original shape easily.

## Claims

1. A device with a bi-directional valve for injecting medicine and drawing body fluid without using a needle having
• a connector (10, 10a, 10b, 10c, 10d, 10e) having
- a top having a center,
- a bottom,
- a plane (11),
- a passage (12) formed through the connector (10, 10a, 10b, 10c, 10d, 10e) and having
a top end opening defined in the plane (11); and
- a sidewall (13) formed on the top of the connector (10, 10a, 10b, 10c, 10d, 10e) and around the top end of the passage (12) and having
a top,
an outer face, and
an inner face,
• a resilient plug (30) mounted on the plane (11) and having
- a top,
- a bottom,
- a neck (31) being cylindrical and having
a diameter,
a top, and
a bottom,
- a flange (32) formed on the top of the neck (31) and having
a top, and
a smooth edge,
- a bottom tube (33) extending downward from the bottom of the neck (31), having a diameter larger than that of the neck (31) and having
a bottom,
an outer surface, and
an inner surface,
- an annular shoulder (34) formed between the neck (31) and the bottom tube (33),
- an inner cavity (35) formed in the neck (31) and communicating with the bottom tube (33), and
• a cap (40) mounted on the resilient plug (30) and having
- a top,
- a bottom,
- an inner surface,
- a threaded neck (42) formed on and extending from the top of the cap (40) and having a top,
- an opening (41) defined in the top of the threaded neck (42) and corresponding to the top of the flange (32) on the resilient plug (30),
- a shoulder (43) mounted between the top of the cap (40) and the threaded neck (42),
**characterized in that**
- multiple holes (36) are formed through the bottom tube (33) near the bottom, and **in that**
- a concave inner surface (45) is defined in the inner surface of the cap (40) at a joint between the cap (40) and the threaded neck (42), and **in that**
- a horizontal constriction (37) is formed on the outer surface of the bottom tube so the resilient plug (30) topples down from the horizontal constriction (37) when the resilient plug (30) is pushed.

2. The device as claimed in claim 1, wherein the device further comprises a protruding ring (20) formed on the plane (11) and around the top end of the passage (12) and the resilient plug (30) is mounted around the protruding ring (20).

3. The device as claimed in claim 1, wherein the device further comprises a protruding ring (20a) mounted on the plane (11) and around the top end of the passage (12) and the resilient plug (30) is mounted around the protruding ring (20a).

4. The device as claimed in claim 2, wherein the connector (10) further comprises multiple passages (14) to connect with tubes.

5. The device as claimed in claim 4, wherein the cap (40) further comprises a mounting ring (44) formed near the bottom on the cap (40) and mounted securely on the sidewall (13) to hold the cap (40) in the connector (10) and around the resilient plug (30).

6. The device as claimed in claim 3, wherein the connectors (10a, 10b, 10c, 10d, 10e) are tubular.

7. The device as claimed in claim 6, wherein the connector (10a) further comprises an inner threaded joint (15) formed under the plane (11) to connect to a tube.

8. The device as claimed in claim 6, wherein the connector (10c) is Y-shape.

## Patentansprüche

1. Vorrichtung mit einem Zweiwegeventil zum Einspritzen von medizinischen Lösungen und Entnehmen von Flüssigkeit ohne Verwendung einer Nadel, die aufweist
• ein Anschlussstück (10, 10a, 10b, 10c, 10d, 10e), das aufweist
- ein Oberteil, das ein Mittelteil aufweist
- ein Unterteil
- eine Ebene (11)
- einen Durchgang (12), der durch das Anschlussstück (10, 10a, 10b, 10c, 10d, 10e) gebildet ist und aufweist
eine Öffnung am oberen Ende, die in der Ebene (11) bestimmt ist; und
- eine Seitenwand (13), die an der Oberseite des Anschlussstücks (10, 10a, 10b, 10c, 10d, 10e) und um das obere Ende des Durchgangs (12) gebildet ist und aufweist
ein Oberteil,
eine Außenfläche und
eine Innenfläche
• einen federnden Stopfen (30), der auf der Ebene (11) montiert ist und aufweist
- ein Oberteil
- ein Unterteil
- einen Hals (31), der zylindrisch ist und aufweist
einen Durchmesser
ein Oberteil und
ein Unterteil
- einen Flansch (32), der oben auf dem Hals (31) gebildet ist und aufweist
ein Oberteil und
eine glatte Kante
- ein unteres Rohr (33), das sich nach unten vom Unterteil des Halses (31) erstreckt, das einen Durchmesser aufweist, der größer ist als der des Halses (31) und aufweist
ein Unterteil
eine Außenfläche und
eine Innenfläche
- eine ringförmige Schulter (34), die zwischen dem Hals (31) und dem unteren Rohr (33) gebildet ist
- einen inneren Hohlraum (35), der in dem Hals (31) gebildet ist und mit dem unteren Rohr (33) in Verbindung steht und
• eine Kappe (40), die auf dem federnden Stopfen (30) montiert ist und aufweist
- ein Oberteil
- ein Unterteil
- eine Innenfläche
- einen Gewindehals (42), der auf dem Oberteil der Kappe (40) gebildet ist und sich von ihr erstreckt und ein Oberteil aufweist
- eine Öffnung (41), die in dem Oberteil des Gewindehalses (42) bestimmt ist und dem Oberteil des Flansches (32) auf dem federnden Stopfen (30) entspricht
- eine Schulter (43), die zwischen dem Oberteil der Kappe (40) und dem Gewindehals (42) montiert ist,
**dadurch gekennzeichnet, dass**
- mehrere Löcher (36) durch das untere Rohr (33) neben dem Unterteil gebildet sind, und **dadurch**, dass
- eine konkave Innenfläche (45) in der Innenfläche der Kappe (40) an einer Verbindung zwischen der Kappe (40) und dem Gewindehals (42) bestimmt ist, und **dadurch**, dass
- eine horizontale Verjüngung (37) an der Außenfläche des unteren Rohres gebildet ist, so dass der federnde Stopfen (30) von der horizontalen Verjüngung (37) herabfällt, wenn der federnde Stopfen (30) gedrückt wird.

2. Vorrichtung nach Anspruch 1, wobei die Vorrichtung überdies einen vorstehenden Ring (20) umfasst, der auf der Ebene (11) und um das obere Ende des Durchgangs (12) gebildet ist, und der federnde Stopfen (30) um den vorstehenden Ring (20) montiert ist.

3. Vorrichtung nach Anspruch 1, wobei die Vorrichtung überdies einen vorstehenden Ring (20a) umfasst, der auf der Ebene (11) und um das obere Ende des Durchgangs (12) montiert ist, und der federnde Stopfen (30) um den vorstehenden Ring (20a) montiert ist.

4. Vorrichtung nach Anspruch 2, wobei das Anschlussstück (10) überdies mehrere Durchgänge (14) für den Anschluss mit Rohren umfasst.

5. Vorrichtung nach Anspruch 4, wobei die Kappe (40) überdies einen Montagering (44) umfasst, der neben dem Unterteil der Kappe (40) gebildet ist und sicher an der Seitenwand (13) montiert ist, um die Kappe (40) im Anschlussstück (10) und um den federnden Stopfen (30) zu halten.

6. Vorrichtung nach Anspruch 3, wobei die Anschlussstücke (10a, 10b, 10c, 10d, 10e) rohrförmig sind.

7. Vorrichtung nach Anspruch 6, wobei das Anschlussstück (10a) überdies eine innere Gewindeverschraubung (15) umfasst, die unter der Ebene (11) für den Anschluss an ein Rohr gebildet ist.

8. Vorrichtung nach Anspruch 6, wobei das Anschlussstück (10) Y-förmig ist.

## Revendications

1. Dispositif comprenant une valve bidirectionnelle pour l'injection de solutions médicales et le prélèvement de fluides sans l'utilisation d'une aiguille présentant
• un raccord (10, 10a, 10b, 10c, 10d, 10e) présentant
- une partie supérieure présentant un centre,
- une partie inférieure,
- un plan (11),
- un passage (12) formé à travers le raccord (10, 10a, 10b, 10c, 10d, 10e) et présentant
une ouverture à l'extrémité supérieure définie dans le plan (11) et
- une paroi latérale (13) formée sur la partie supérieure du raccord (10, 10a, 10b, 10c, 10d, 10e) et autour de l'extrémité supérieure du passage (12) et présentant
une partie supérieure,
une surface externe et
une surface interne,
• un bouchon résilient (30) monté sur le plan (11) et présentant
- une partie supérieure,
- une partie inférieure,
- un col (31) qui est cylindrique et présente
un diamètre,
une partie supérieure et
une partie inférieure,
- une collerette (32) formée sur la partie supérieure du col (31) et présentant une partie supérieure et
un bord lisse,
- un tube inférieur (33) s'étendant vers le bas depuis la partie inférieure du col (31), présentant un diamètre supérieur à celui du col (31) et présentant
une partie inférieure,
une surface externe et
une surface interne,
- un épaulement annulaire (34) formé entre le col (31) et le tube inférieur (33),
- une cavité interne (35) formée dans le col (31) et communicant avec le tube inférieur (33), et
• un capuchon (40) montée sur le bouchon résilient (30) et présentant
- une partie supérieure,
- une partie inférieure,
- une surface interne,
- un col fileté (42) formé sur et s'étendant depuis la partie supérieure du capuchon (40) et présentant une partie supérieure,
- une ouverture (41) définie dans la partie supérieure du col fileté (42) et correspondant à la partie supérieure de la collerette (32) sur le bouchon résilient (30),
- un épaulement (43) monté entre la partie supérieure du capuchon (40) et le col fileté (42),
**caractérisé en ce que**
- plusieurs trous (36) sont formés à travers le tube inférieur (33) près de la partie inférieure et **en ce que**
- une surface interne concave (45) est définie dans la surface interne du capuchon (40) à une jonction entre le capuchon (40) et le col fileté (42) et **en ce que**
- un rétrécissement horizontal (37) est formé sur la surface externe du tube inférieur de sorte que le bouchon résilient (30) tombe depuis le rétrécissement horizontal (37) quand on appuie sur le bouchon résilient (30).

2. Dispositif selon la revendication 1, dans lequel le dispositif comprend en outre un anneau faisant saillie (20) formé sur le plan (11) et autour de l'extrémité supérieure du passage (12) et le bouchon résilient (30) est monté autour de l'anneau faisant saillie (20).

3. Dispositif selon la revendication 1, dans lequel le dispositif comprend en outre un anneau faisant saillie (20a) monté sur le plan (11) et autour de l'extrémité supérieure du passage (12) et le bouchon résilient (30) est monté autour de l'anneau faisant saillie (20a).

4. Dispositif selon la revendication 2, dans lequel le raccord (10) comprend en outre plusieurs passages (14) pour un raccord avec des tubes.

5. Dispositif selon la revendication 4, dans lequel le capuchon (40) comprend en outre un anneau de montage (44) formé près de la partie inférieure sur le capuchon (40) et monté solidement sur la paroi latérale (13) pour retenir le capuchon (40) dans le raccord (10) et autour du bouchon résilient (30).

6. Dispositif selon la revendication 3, dans lequel les raccords (10a, 10b, 10c, 10d, 10e) sont tubulaires.

7. Dispositif selon la revendication 6, dans lequel le raccord (10a) comprend en outre un joint fileté interne (15) formé sous le plan (11) pour un raccord avec un tube.

8. Dispositif selon la revendication 6, dans lequel le raccord (10c) est en forme de Y.
